# EUROPEAN PATENT APPLICATION

(11) **EP 1 595 534 A1**
(43) Date of publication of application: **16.11.2005**
(21) Application number: 04076424.3
(22) Date of filing: 13.05.2004
(51) Int. Cl.: A61K 9/22, A61K 47/36

(54) **Gel composition comprising charged polymers**

(71) Applicant: Universiteit Utrecht Holding B.V., 3584 CK Utrecht (NL)
(72) Inventor: van Tomme, Sophie Rolande, 3555 RA Utrecht (NL); van Steenbergen, Marinus Jacob, 6716 DM Ede (NL); van Nostrum, Cornelis Franciscus, 5251 CV Vlijmen (NL); Hennink, Wilhelmus Everhardus, 2743 CZ Waddinxveen (NL)
(74) Representative: Winckels, Johannes Hubertus F.

(57) **Abstract**

Aqueous gel compositions are disclosed which are useful as drug carriers for controlled delivery applications, such as for parenteral depot formulations. The gels comprise ionically charged microparticles, and preferably mixtures of oppositely charged microparticles. The microparticles can be loaded with active compounds, such as therapeutic peptides and proteins. The gels can be prepared by combining oppositely charged microparticles in the presence of water.

## Description

The present invention relates to pharmaceutical compositions in the form of gels. More in particular, it relates to aqueous gels which are particularly suitable for parenteral administration and/or which are capable of providing slow, sustained or controlled release of active compounds. Further, these gels are particularly suitable for tissue engineering. In further aspects, the invention relates to uses of such gels and methods of preparing them. Furthermore, kits are provided from which pharmaceutical gel compositions can be prepared.

A gel may be defined by virtue of its rheological properties. A macroscopic gel which can be used as a pharmaceutical dosage form is semisolid: it behaves like a solid upon the exertion of low shear force, but above a certain force threshold, the so-called "yield point", it behaves like a viscous fluid. It is this definition according to which the term "gel" is used in the present description and appending claims.

Aqueous gels have been known as pharmaceutical or cosmetic dosage form for many years. Originally, they have been mostly used for topical administration, such as for dermal, ophthalmic, vaginal, oromucosal, or rectal application. Within the field of pharmaceutical and cosmetic formulation science, a more narrow definition of a gel has also emerged, according to which gels are typically transparent preparations comprising a coherent liquid phase and a three-dimensional network of a colloidal solid which imparts resistance to flow. According to this concept, rheologically similar systems with a different physical structure, such as ointments and pastes, would not be considered gels.

Another related physicochemical concept is that of hydrogels. Hydrogels form an important class of materials for tissue engineering and for the sustained or otherwise controlled release of pharmaceutically active compounds such as therapeutic proteins. Hydrogels are three-dimensional polymeric networks made by chemical or physical cross-linking of hydrophilic polymers. In chemically cross-linked gels, the polymers are connected primarily by covalent bonds. In physically cross-linked gels, the network is formed by physical interactions between different polymer chains, such as by electrostatic interactions. Accordingly, the respective hydrogels are sometime termed physical hydrogels, whereas chemically cross-linked hydrogels are also called chemical hydrogels. Within the context of the present invention, chemical or physical hydrogels are not necessarily identical with macroscopic gels as defined above. Only if they have the typical rheological behaviour of gels as referred to herein-above, such hydrogels may at the same time represent macroscopic gels. On the other hand, even hydrogels which do not have the rheological behaviour of gels, i.e. which represent solid materials, may still be components or constituents of macroscopic gels.

Parenteral dosage forms with slow drug release properties have been developed to answer the need for improving the therapeutic use of drug substances which cannot be administered orally due to their physicochemical properties, and which have a relatively short half life because of which they have to be injected frequently. Frequent injections are uncomfortable to patients, and if the injections have to be given by physicians or nurses, they are also rather costly. The experience of discomfort and pain may result in patient incompliance and jeopardise the success of the therapy.

The number of drug substances which cannot be administered by the preferred oral route is presently increasing, primarily as a consequence of the recent advances of biotechnological research in the pharmaceutical area, which has lead to an increased number of highly potent peptide and protein drugs. Perhaps with the exception of some smaller peptides, however, these compounds are relatively unstable in gastrointestinal fluids and, more importantly, too large and hydrophilic as molecules to become absorbed through the intestinal mucosa to a substantial extent. For some of these drug substances, injectable or implantable controlled release formulations are being developed in order to lower the dosing frequency and thus reduce patient discomfort, and achieve a higher level of compliance and therapeutic success.

Parenteral controlled release dosage forms are usually in the form of macroscopic, solid single- or multiple-unit implants (such as polymeric rods and wafers), microparticle suspensions, and more recently also gels, including *in situ* forming gels. Drug-loaded solid implants are available as non-degradable polymeric, ceramic or metal devices which have to be surgically removed after the designated period of drug action, or as biodegradable polymeric forms which require no removal. An example for a non-degradable implant is Bayer's Viadur®, which releases the peptide drug leuprolide over a period of one year. An example for a biodegradable implant is AstraZeneca's Zoladex® , which is a polymeric rod capable of releasing the peptide drug goserelin over periods of one and three months, respectively.

A major disadvantage of implants is that they require very large needle diameters to be inserted, or in some cases even surgical incisions. Without premedication with a local anaesthetic, the implantation procedure would not be considered acceptable by a large number of patients. On the other hand, local anaesthesia can ensure that the procedure is painless.

Shortly after the market introduction of the first biodegradable implants, controlled release microparticles became available, such as Takeda's Lupron® Depot formulations, which release leuprolide over periods of one, three, and four months, respectively. In order to inject such microparticles, they have to be suspended in an aqueous carrier. For stability reasons, however, depot microparticles cannot usually be stored as an aqueous suspension, but they have to be reconstituted from a dry powder.

Various designs of drug-loaded microparticles and methods for their preparation are described in E. Mathiowitz et al., Microencapsulation, in: Encyclopedia of Controlled Drug Delivery (ed. E. Mathiowitz), Vol. 2, (1999) 493-546, John Wiley & Sons, which is incorporated herein by reference.

Considering the required needle sizes for injecting microparticle suspensions (typically gauche 19 to 22), there is still a large difference to the relatively painless administration of a solution for injection. Smaller needle diameters are theoretically feasible for some microparticle preparations, but they bring about the risk of needle clogging in case the reconstitution is not carried out very carefully.

To overcome these problems, drug delivery scientists have in recent years begun to develop injectable gels which are capable of forming subcutaneous or intramuscular depots. In one of the concepts, gel formulations are designed which are highly shear thinning and thixotropic. By applying shear force prior to administration, the viscosity of these gels is substantially reduced, allowing for injection with a relatively small needle, whereas the gel strength is recovered slowly after administration. According to another concept, liquid compositions are formulated which, after administration, form gels in response to changes of their environment, such as pH, temperature, ionic strength. According to a third approach, liquid polymer formulations comprising a non-aqueous solvent are injected. Upon administration, the solvent diffuses away from the injection site, which leads to the precipitation of polymeric particles or to the formation of a gel.

Biodegradable injectable gels have been discussed in detail by A. Hatefi et al., Journal of Controlled Release 80 (2002), 9-28, which document is incorporated herein by reference.

Notwithstanding the recent progress in the design and manufacture of injectable gel formulations for controlled drug delivery, there remains a need for further improved gels, compositions, and preparation methods which overcome one or more of the disadvantages associated with presently available gels.

It is an object of the invention to provide improved pharmaceutical gels which are suitable for parenteral administration, convenient to use, and which have properties allowing their injection with acceptable needle sizes. It is another object of the invention to provide gels comprising polymeric drug carriers which are safe and allow the tuning of release rates over periods of days, weeks, and months. In a further aspect, it is an object of the invention to provide kits and methods for making such gels. Further objects will become apparent by reading the following description.

According to the invention, an aqueous gel composition is provided which comprises a first type of microparticles comprising a cationic polymeric material, and a second type of microparticles comprising an anionic polymeric material. Optionally, the gel further comprises an active compound.

In another aspect, the invention provides an aqueous gel composition comprising an active compound and ionically charged polymeric microparticles, wherein the microparticles are suspended in a solution or dispersion of an ionic polymer whose charge is opposite to that of the microparticles.

The microparticles which are present in the gel are ionically charged. They are preferably based on polymers, preferably biodegradable polymers, which are suitable for pharmaceutical applications. At least a fraction of the polymer molecules which form the microparticles bear at least one ionic charge.

In order to form an aqueous gel, the composition must contain a significant amount of water. The viscoelastic properties of the gel are believed to be imparted by the ionic interactions occurring between the two types of microparticles with opposite charges, or between charged microparticles and the oppositely charged polymer which is dissolved or dispersed in the continuous aqueous phase of the gel.

In another aspect, the invention provides a kit for the preparation of aqueous gel compositions. Such a kit may comprise a dry solid component and an aqueous liquid for reconstituting the gel composition, wherein the solid component comprises the active compound. Optionally, the dry solid component also comprises at least one type of charged microparticles.

In a further aspect, the invention provides a method for making an aqueous gel. In one of the preferred embodiments, the method comprises the step of combining positively charged polymeric microparticles with negatively charged polymeric microparticles in the presence of water. In another principal embodiment, the method comprises the step of combining ionically charged polymeric microparticles with an ionic polymer whose charge is opposite to that of the microparticles, in the presence of water.

Furthermore, the invention discloses the use of aqueous gel compositions comprising an active compound, a first type of microparticles comprising a cationic polymer, and a second type of microparticles comprising an anionic polymer, as slow, sustained or otherwise controlled release carriers for the parenteral delivery of active compounds.

In a first principal aspect, the invention provides an aqueous gel composition. The composition comprises a first type of microparticles comprising a cationic polymeric material, and a second type of microparticles comprising an anionic polymeric material. At neutral, that is physiological, pH, the first type of microparticles which comprise a cationic polymeric material are positively charged, whereas the second type of microparticles which comprise an anionic polymeric material are negatively charged.

As used herein, a gel is a semisolid material which behaves like a solid upon the exertion of low shear force, and like a viscous fluid when the shear force exceeds a threshold which is defined as the yield point. In other words, a gel is a system with a finite, usually rather small, yield stress.

Preferably, the gel composition further comprises an active compound. An active compound is any chemical or biological substance or mixture of substances which is useful for the diagnosis, prevention or treatment of diseases, symptoms, and other conditions of the body, or for influencing a body function. To be an active system, the gel composition comprises at least one, and optionally two or more of such active compounds.

Preferred active compounds are those which are used in chronical or long-term treatment regimen and/or which have a low oral bioavailability, such as hormones, growth factors, hormone antagonists, antipsychotics, antidepressants, cardiovascular drugs, and the like. In another aspect, a preferred class of active compounds is that of peptides and proteins, in particular proteins, which can be delivered effectively with the gel compositions of the invention, providing drug release over extended time periods, thus eliminating the need for the frequent injection of these compounds.

Among the preferred peptides and proteins are erythropoetins, such as epoetin alpha, epoetin beta, darbepoetin, haemoglobin raffimer, and analogues or derivatives thereof; interferons, such as interferon alpha, interferon alpha-2b, PEG-interferon alpha-2b, interferon alpha-2a, interferon beta, interferon beta-1a and interferon gamma; insulins; antibodies, such as rituximab, infliximab, trastuzumab, adalimumab, omalizumab, tositumomab, efalizumab, and cetuximab; blood factors such as alteplase, tenecteplase, factor VII(a), factor VIII; colony stimulating factors such as filgrastim, pegfilgrastim; growth hormones such as human growth factor or somatropin; interleukins such as interleukin-2 and interleukin-12; growth factors such as beclapermin, trafermin, ancetism, keratinocyte growth factor; LHRH analogues such as leuprolide, goserelin, triptorelin, buserelin, nafarelin; vaccines, etanercept, imiglucerase, drotrecogin alpha.

Other preferred active compounds are polysaccharides and oligo- or polynucleotides, DNA, RNA, iRNA, antibiotics, and living cells. Another class of preferred active compounds comprises drug substances acting on the central nervous system, even if they are small molecules and orally bioavailable, for example, risperidone, zuclopenthixol, fluphenazine, perphenazine, flupentixol, haloperidol, fluspirilen, quetiapine, clozapine , amisulprid , sulpirid, ziprasidon, etc.

Alternatively, the active compound may be a native living cell, a modified cell, or a plurality of cells. Encapsulated or immobilised cells can be injected or implanted to replace physiological functions which are absent, or insufficiently present, in a patient due to a specific disease or condition. For example, diabetes patients could be treated with gel-encapsulated Langerhans cells which can produce and secrete insulin. In this application, both the living cells and the insulin could be considered as the active compound.

Microparticles are defined herein as substantially solid or semisolid particles having a weight- or volume average diameter in the region of about 0.1 to about 1.000 µm, but usually of about 1 to about 500 µm, regardless of their composition, geometrical shape, or internal structure. For example, spherical microparticles, which are often referred to as microspheres or nanospheres, are included in the term microparticles, just as capsular structures, such as micro- or nanocapsules. Several other synonyms may exist to describe microparticles as defined above. A suitable method for determining the particle size is described in the working examples herein-below.

A polymer is defined by IUPAC nomenclature as a substance composed of macromolecules. Macromolecules or polymer molecules, in turn, are individual molecules of high relative molecular mass, the structure of which essentially comprises the multiple repetition of units derived, actually or conceptually, from molecules of low relative molecular mass. In common language, however, the term polymer is often used for both the substance and the macromolecules of which a polymeric substance is composed. It is this broader definition which is used herein, i.e. the term can refer to the substance or to a polymeric molecule or macromolecule, as applicable and evident from the respective context.

As used herein, a polymeric material may refer to a polymer, a mixture of polymers, a cross-linked polymer, mixtures thereof, or to polymeric networks. Often, a polymeric material is simply referred to as a polymer.

Consequently, a cationic polymeric material, as used herein, refers to a polymeric material composed of optionally cross-linked macromolecules having one or more elementary charges of the proton, or to such a macromolecule itself. An anionic polymeric material is a macromolecule, or a polymeric material composed of optionally cross-linked macromolecules, having one or more elementary charges of the electron.

An aqueous gel composition is also defined by the presence of water, usually of substantial amounts of water relative to the total mass of the composition. In a typical aqueous gel, the water forms a continuous phase in which the solid components which impart the gel strength to the system are dispersed. If the dispersion is colloidal, such as in a gelatin gel, such system could be considered monophasic, as no interphase can be determined. The gel compositions of the present invention, however, represent systems having at least two phases, i.e. a coherent aqueous phase and a dispersed, discontinuous, or incoherent solid or semisolid phase comprised of microparticles. Depending on the nature of the active compound and other optional constituents, the compositions may comprise even more phases, such as a dispersed liquid phase or further dispersed solid phases.

The anionic and/or cationic microparticles present in the gel composition may be composed of various types of materials, such as organic or inorganic materials with poor water solubility, e.g. lipids, waxes, salts, or polymers. In a preferred embodiment, the microparticles are predominantly based on polymeric components, such as insoluble polymers, or hydrophilic polymers which have been cross-linked to form a hydrogel.

As used herein, hydrogels are three-dimensional polymeric networks made by chemical or physical cross-linking of hydrophilic polymers. In chemically cross-linked gels, the polymers are connected primarily by covalent bonds. In physically cross-linked gels, the network is formed by physical or physicochemical interactions between different polymer chains. Accordingly, the respective hydrogels are sometimes termed physical hydrogels, whereas chemically cross-linked hydrogels are also called chemical hydrogels. For the avoidance of confusion it should be emphasised that, within the context of the present invention, chemical or physical hydrogels are not necessarily identical with macroscopic gels as defined above. Only if they have the typical rheological behaviour of gels, such hydrogels may at the same time represent macroscopic gels. On the other hand, hydrogels are preferred materials from which the microparticles are made, which are present in the gel compositions of the invention.

Hydrogel microparticles can be prepared in various different ways. Particles from physically cross-linked hydrophilic polymers can e.g. be prepared from polyvinyl alcohol or polysaccharides such as certain dextrans by crystallisation. Such microparticles and methods for their preparation are, for example, described in WO 02/17884. Another example of physical cross-linking is the formation of stereocomplexes as described in WO 00/48576. The disclosure of both references is incorporated herein by reference for the preparation of hydrogels which are also useful in the present invention. Furthermore, physically cross-linked hydrogels can be prepared from polyelectrolytes such as gelatin, alginates, xanthan gum, carrageenan, albumin, or chitosan, by ionically cross-linking these with oppositely charged multivalent ions, such as calcium and citrate ions, or polyelectrolytes.

Chemically cross-linked hydrogel microparticles can be prepared, for example, from hydrophilic polymers which have been modified to carry polymerisable groups, such as ethylenically unsaturated groups which can be reacted with each other by radical polymerisation. An example of a physiologically acceptable hydrophilic polymer which has been modified into prepolymers (polymers which are further polymerisable, i.e. which can act like monomers in polymerisation reactions) is dextran. Dextrans with various types of acrylic or methacrylic groups have been prepared and characterised, such as described in the documents WO 98/00170, WO 98/22093, WO 01/60339, and WO 03/035244, the disclosures of which are incorporated herein by reference for the description of dextran and its derivatives.

In a preferred embodiment, the microparticles present in the gel composition of the invention are based on a dextran derivative, such as dextran modified with hydroxyethyl methacrylic groups (dextran hydroxyethyl methacrylate, dexHEMA), in which the hydroxyethyl methacrylic groups are attached to the dextran backbone via carbonate linkers. In a second preferred embodiment, the microparticles contain dextran with oligomeric, hydrolysable side chains composed of lactate and/or glycolate units, wherein the side chains bear hydroxyethyl methacrylic groups.

These chemical hydrogels degrade by hydrolysis in a physiological environment, such as in interstitial fluid. In fact, it is preferred that the microparticles present in the gel composition of the invention are biodegradable in the sense that they are hydrolysable under physiologic conditions. In this context, hydrolysability refers to hydrolysis rates which lead to degradation and elimination within periods of time which are typically considered useful for parenteral controlled release applications, such as over one or several days, weeks, months, or a year. Preferably, degradation and hydrolysis occurs by chemical hydrolysis only, without requiring the presence of certain enzymes. This degradation behaviour can be achieved by preparing the microparticles from hydrolysable polymers such as dexHEMA.

For the avoidance of misunderstandings, hydrolysability does not mean that the hydrogel, or the microparticles, must degrade into the respective monomeric units. It is sufficient that hydrolysis, or degradation, leads to soluble molecular species which can be eliminated from the organism, such as by renal excretion.

Ionically charged microparticles are obtained when at least a fraction of the constituents of the microparticles are ionically charged. In the case of polymeric microparticles, some or all of the polymer molecules of the microparticles are charged. In one of the preferred embodiments, only a fraction of the polymer molecules are charged, such as about 1 to about 60 wt.-% of the polymer molecules of the microparticles. In another embodiment, about 5 to about 35 wt.-%, and preferably about 10 to about 20 wt.-% of the polymer molecules are ionically charged. In the case of polymeric materials representing cross-linked polymers or polymeric networks, these figures should be applied to the percentage of macromolecular main chains participating in the network.

In the present description and the appending claims, a "charged polymer" is a polymer comprised of monomers wherein the molar ratio of the charged monomers to the non-charged monomers is between 1/100 to 100/1, preferably between 1/20 to 20/1, more preferably between 1/10 to 10/1, and most preferably between 1/4 to 4/1. In this ratio "charged monomer" means a monomer having a positive or negative charge in the polymer at the pH in the system wherein the polymer is used. Should there be different monomers having positive and negative charges than the difference between these values need to be used. Non-charged monomers lead to a non-charged building unit in the polymer.

For instance, in the preferred embodiments wherein polymers are prepared using hydroxyethyl methacrylate (HEMA), and dimethylamino ethylmethacrylate (DMAEMA) (for cationic polymers) or HEMA and methacrylic acid (MAA) (for anionic polymers), very suitable results are obtained when the molar ratios of HEMA/DMAEMA and HEMA/MAA and between 0,25 and 1.5; preferably about 0.5.

If dextran derivatives are used to prepare the microparticles, cationic charges may be achieved by the incorporation of cationically modified dextrans, such as DEAE-dextran. Alternatively, positively charged polyelectrolytes may be used, such as chitosan, certain types of gelatin, protamin, poly-spermidin, positively charged polyphosphazenes and polystyrenes, or polydimethylaminoethyl methacrylate (pDMAENU). One of the presently preferred monomeric units to induce a cationic charge to the microparticles is DMAEMA.

Anionic charges can be achieved by the incorporation of anionically modified dextrans, or by other anionic polymers, such as carboxymethyl cellulose, carboxymethyl starch, alginic acid, polyacrylic acid, polymethacrylic acid, certain types of gelatin, hyaluronic acid, DNA, negatively charged polyphosphazenes, or carrageenan.

Presently preferred is the use of acrylic or methacrylic acid as anionic species, or dimethylaminoethyl methacrylate as cationic species, in combination with neutral modified dextrans such as dexHEMA. Using these combinations, the ionic monomeric units will participate in the polymerisation or cross-linking reaction of dexHEMA which leads to the formation of a three-dimensional polymeric network.

In one of the preferred embodiment, the active compound present in the gel composition is incorporated in the microparticles; and preferably, at least a fraction of the microparticles present in the composition is loaded with active compound. If two or more active compounds are incorporated in the composition, at least one of them, and preferably all of them, are accommodated within microparticles. Based on this embodiment, release rates can be tailored through adjusting the hydrolysability of the polymers forming the microparticles. For example, if the active compound is a macromolecular substance, such as a protein, and the microparticles are hydrogels representing water-swollen, porous, three-dimensional polymeric networks, drug release is likely to take place primarily by degradation and erosion of the hydrogel, as the pores of the non-degraded hydrogel will typically be too small to allow for drug release by diffusion.

It is however possible and in specific situations desirable to incorporate some of the active compound into the aqueous gel phase instead of within the microparticles. For instance, if a more rapidly releasable dose fraction is needed for an initial therapeutic effect such as a quick or pronounced onset of action, a specified dose fraction may be incorporated in such non-retarded state.

Often, however, the active compound is incorporated within the microparticles present in the compositions. This means for the present invention that at least about 90 wt.-% of the incorporated drug dose is incorporated in microparticles, preferably at least 95 wt.-%. Even more preferably, all or substantially all of the active compound is located in the microparticles, at least after the manufacture of the gel composition or of a kit from which the gel composition can be prepared shortly before administration.

While the microparticles play a major role in controlling the release rate of the incorporated active compound, they also determine the rheological behaviour of the gel to a large degree. The higher the charge density of the oppositely charged particles, the higher are the electrostatic interactions between them which impart resistance to gel deformation by shear force.

Another factor influencing the rheological properties of the gel is its total particle content. Depending on the size, chemical nature, and surface charge density of the particles, but also on the composition of the aqueous continuous phase of the gel, a threshold particle content must be exceeded to allow particle association and to impart gel properties to the system. On the other hand, a very high particle content may lead to solid systems which have no yield point, or whose yield point is too high for the applications envisioned herein. Typically, a useful particle content of the composition is in the region of about 1 to about 70 wt.-%. More preferably, it is about 5 to about 50 wt.-%. In another embodiment, the particle content is about 10 to about 30 wt.-%. Presently, most preferred is a particle (or solid) content of about 10 to about 20 wt.-%.

The size of the microparticles will also influence the rheological properties. Primarily, however, the particle size should be selected to enable convenient administration of the gel composition. For example, if the composition is used as an injectable depot formulation for the controlled release of an active compound after intramuscular or subcutaneous administration, it should be injectable with needles small enough to cause no or little pain when inserted. Preferably, the gel composition of the invention is adapted to be capable of being administered with a needle of 17 gauge or higher, and more preferably with a needle of 20 gauge or higher, and even more preferably with a needle of 22 gauge, 24 gauge, 26 gauge, or higher.

As used herein, the capability of being administered refers to rheological properties which allow the injection with the specified needle type without requiring an injection force of more than about 25 N. More preferably, the rheological properties are adapted, and a needle size selected, to enable injection with a force of no more than about 20 N, and even more preferably with an injection force of no more than about 15 N, to allow the administration also to be performed by physicians, nurses, or patients who are not particularly sinewy.

To prevent the microparticles from clogging small needles, their weight or volume average particle size should be selected in the range of up to about 100 µm. More preferably, the average particle size should be in the region of about 1 to about 50 µm. Presently most preferred are average particle sizes in the region of about 2 to about 20 µm, which allow the use of very fine hypodermic needles for injection.

The gel composition, and in particular the particle content, should generally be selected to obtain a gel with a yield point in the region of about 5 to about 300 Pa, preferably of 10 to 250 Pa, and more preferably in the region of about 25 to about 200 Pa, wherein the yield point is determined as described in example 4.

The viscous behaviour of the gel composition above the yield point should preferably be linear, or Newtonian, or it should have shear-thinning characteristics, becoming less viscous upon increased shear stress. For administration purposes, shear-thinning behaviour above the yield point is rather useful as it means that the composition can be injected rather rapidly without applying too much force. In another aspect, the composition is preferably shear thinning and at the same time thixotropic, which means that after applying shear stress it takes some time for the original gel strength to recover. Without wishing to be bound by a particular theory, it is believed that the electrostatic interactions between the oppositely charged microparticles in the gel are at least partially disrupted upon applying shear stress above the yield point, and that returning from the state of flow after removal of shear stress means that the individual microparticles first have to associate again to form a gel.

More important than the viscous properties of the composition above the yield point is that it sets again when the shear stress is removed. In other words, the gel must not be susceptible to rheodestruction, which refers to the irreversible liquefaction of some gels through shearing.

The gel compositions of the invention are capable of releasing active compounds slowly and over predetermined periods of time, such as over days, weeks, or months. Therefore, they represent useful carriers for the delivery of drug substances, especially for parenteral administration. Slow release, as used herein, encompasses all types of modified release profiles, such as controlled release, sustained release, prolonged release, delayed release, pulsed release etc. Pulsed release, for example, may be accomplished by incorporating some of the active ingredients within the aqueous gel phase of the composition, and the remaining dose fraction within microparticles, which is released in a second pulse of drug release.

In a further embodiment, the active compound may be incorporated in the form of drug-loaded colloidal carriers, such as nanoparticles, nanocapsules, liposomes, lipoplexes, lipid complexes, iscoms, polyplexes, solid lipid nanoparticles, virosomes, or drug conjugates.

The gel compositions can be adapted for topical, oral, rectal, vaginal, or ophthalmic administration; preferably, however, they are adapted for parenteral administration. As used herein, parenteral administration includes any invasive route of administration, such as subdermal, intradermal, subcutaneous, intramuscular, locoregional, intratumoral, intraperitoneal, interstitial, intralesional, with some less preference in the context of this invention also intravenous, intraarterial etc. The most preferred routes of administration of the gel compositions are subcutaneous, intramuscular, and intratumoral.

When the invention is used for tissue engineering applications, the sites of injection or implantation may of course differ widely, depending on the specific tissue or organ whose function is to be replaced or augmented. As mentioned above, in these applications it is the living cells that take the place of the active compound.

Being adapted for parenteral administration also means that such gel compositions are formulated and processed to meet the requirements of parenteral dosage forms. Such requirements are, for example, outlined in the major pharmacopoeias. In one aspect, the composition, or its premixes or the kits from which the composition is made prior to administration, must be sterile. In another aspect, the excipients must be selected to be safe and tolerable for parenteral administration. In a further aspect, the compositions are formulated to be relatively isotonic (or isoosmotic), such as in the region of about 150 to 500 mOsmol/kg, and preferably in the region of about 250 to 400 mOsmol/kg. Furthermore, the pH should be approximately in the physiological range in order to avoid pain and local intolerance upon injection. Preferably, the pH of the composition is in the region of about 4 to 8.5, and more preferably in the region of about 5.0 to 7.5.

Further excipients may be incorporated in the gel composition as needed, such as stabilisers, bulking agents, matrix forming agents, lyophilisation aids, antioxidants, chelating agents, preservatives, solvents, cosolvents, surfactants, osmotic agents, acidic or alkaline excipients for adjusting the pH, *etc.*

In another embodiment, the invention provides an aqueous gel composition comprising an active compound and ionically charged polymeric microparticles, wherein the microparticles are suspended in a solution or dispersion of an ionic polymer whose charge is opposite to that of the microparticles. In other words, these gel compositions are also based on ionically charged microparticles, but only on one type of particles which interact electrostatically with an oppositely charged polymer - preferably a polyelectrolyte - which is not in the form of particles. Still, the interaction leads to the formation of a macroscopic, aqueous gel if the participating polymers and their content in the composition are selected appropriately. In general terms, the same features and embodiments as outlined above for gel compositions comprising oppositely charged microparticles also apply to gel compositions in which only one charged polymer is in the form of particles.

The gel composition of the invention which comprises two types of microparticles which are oppositely charged can be prepared by a method which comprises the step of combining positively charged microparticles with negatively charged microparticles in the presence of water.

In one embodiment, anionically charged microparticles and cationically charged particles are prepared separately. Preferably, one or both types of microparticles are loaded with the active compound during or after the preparation of the particles. Usually, the formation of the particles and/or the loading with the drug substance will take place in a liquid system, such as in a solution, suspension, or emulsion. Subsequently, several options for combining the two types of microparticles in the presence of water exist. According to a first option, portions of aqueous suspensions of each type of microparticles are combined and mixed, leading to spontaneous gel formation. Alternatively, each type of microparticles can be separately dried. The dry powders can then be mixed, and the mixture hydrated with an aqueous liquid. Yet another option is to combine and/or mix an aqueous suspension containing one type of microparticles with a dry component, such as a powder, comprising the other type of microparticles.

The gel composition of the invention which comprises only one type of charged microparticles can be prepared by methods which comprise the step of combining ionically charged polymeric microparticles with an ionic polymer whose charge is opposite to that of the microparticles, in the presence of water.

Preferably, the microparticles are loaded with the active compound during or after their preparation. Subsequently, an aqueous suspension of the microparticles is combined with an aqueous solution or dispersion of the oppositely charged polymer. Alternatively, the microparticles are provided in dry form, such as in form of a powder, and combined with a solution or dispersion of the oppositely charged polymer. According to a further option, a dry mixture of the microparticles and the oppositely charged polymer is prepared, which is subsequently hydrated with an aqueous liquid.

Active compounds selected according to the preferences disclosed above are, however, very often unstable in an aqueous environment. It may therefore be necessary in order to achieve a commercially acceptable shelf-life to store the gel composition of the invention in a dry state from which it can be reconstituted with an appropriate aqueous carrier prior to administration. Preferably, a kit is provided to the end user, e.g. the patient, nurse, pharmacists, or physician, which comprises all constituents needed to prepare the gel composition.

It is recommended that the kit comprises as few separately packaged components as needed to prepare the gel composition. Preferably, the kit comprises a dry, solid component, and an aqueous liquid for reconstituting the gel. The dry, solid component can be in the form of a powder, such a lyophilised powder, or in the shape of a porous single unit as it sometimes results from lyophilising an aqueous suspension, depending on the excipients. As it is preferred according to the invention that the active compound is incorporated into the microparticles, or at least into one type of microparticles of the gel, the solid component will usually comprise drug-loaded microparticles.

In one embodiment, the solid component of the kit comprises both types of microparticles, i.e. anionically and cationically charged, respectively, wherein both types of microparticles are loaded with the active ingredient(s), and optionally, one or more further excipients, e.g. one or more stabilisers, bulking agents, matrix forming agents, lyophilisation aids, antioxidants, preservatives, surfactants, osmotic agents, acidic or alkaline excipients for adjusting the pH, *etc.* The solid component of the kit is preferably sterile and packaged in a vial, ampoule, or in a syringe.

The aqueous liquid comprises predominantly water. Optionally, it may contain further excipients, such as one or more osmotic agents (e.g. sodium chloride, a sugar, or sugar alcohol), or surfactants (e.g. Tween® 80), or excipients to adjust the pH. In some cases, it may be advantageous to also accommodate one type of microparticles in the liquid component of the kit, e.g. when that type of microparticles reduces the shelf life of the solid component when incorporated therein. In this context, aqueous means that water is either the only or the most abundant liquid substance in the liquid component. In other words, other liquids may optionally be present if pharmaceutically desirable, such as ethanol, glycerol, propylene glycol, polyethylene glycol, triacetin, etc.

The liquid component can be packaged in a sterile bottle, vial, ampoule, or in a separately sealed chamber of a syringe which also holds the dry, solid component of the kit. Preferably, the primary packages of the kit each hold the quantity of solid or liquid component which is needed for preparing one single dose unit.

Further embodiments will become obvious from the following examples which illustrate the invention in some of its major aspects, without limiting the scope thereof.

### Example 1: Preparation of cationically charged microparticles.

Cationically charged microspheres based on dextran hydroxyethyl methacrylate (dexHEMA) and dimethylaminoethyl methacrylate (DMAEMA) were prepared as follows. DexHEMA was prepared as described in WO-98/00170. The degree of substitution DS, i.e. the number of HEMA groups per 100 glucopyranose units, was 6. DMAEMA was obtained from a commercial supplier.

Aqueous solutions of polyethylene glycol (PEG, 40 % (w/w)) and dexHEMA (20 % (w/w)) were prepared in Hepes buffer (100 mM pH 7). PEG, dexHEMA and buffer solution, 197.6 g, 18.3 g and 284.1 g, respectively were transferred into a glass cylinder. Subsequently, 12.5 mmol of DMAEMA was added to the two-phase system (molar ratio HEMA/DMAEMA=0.53). The liquid was flushed with nitrogen and intensively mixed using an IKA Ultra-Turrax® T 25 basic (30 minutes, 11,000 rpm). In this way, a water-in-water emulsion was created whose dispersed phase is rich in dexHEMA and DMAEMA. The emulsion was allowed to stabilise for 15 minutes.

Next, the methacrylic groups of the polymers were polymerised to form a three-dimensional polymeric network. A solution of N,N,N',N'-tetramethylethylenediamine (TEMED, 10 mL, 20 % v/v, adjusted to pH 7 with 4 M HCl) and a solution of potassium peroxodisulfate (KPS, 18 mL, 50 mg/mL), both freshly prepared, were added to the mixture. The emulsified particles were allowed to polymerise for 30 minutes at ambient temperature. The cross-linked particles were collected and purified by multiple washing and centrifugation steps (thrice with reversed osmosis water, 15 minutes, 3000 rpm). The equilibrium water content was 70 wt.-%. For storage, the microparticles were lyophilised.

For determining the particle size distribution, the lyophilised microspheres were resuspended in Isoton II (Beckman Coulter GmbH, Germany), and ultrasound was used to homogenise the dispersion. The measurement was carried out at room temperature using a Coulter Counter Multisizer® 3 with a 100-µm orifice. The calibration of the instrument was performed using 20-µm latex beads (Coulter CC size standard L20). In result, the mean volume diameter was 7.5 µm, and about 90 % of the particles were smaller than 12.5 µm.

### Example 2: Preparation of anionically charged microparticles.

Anionically charged microspheres based on dextran hydroxyethyl methacrylate (dexHEMA) and methacrylic acid (MAA) were prepared essentially as described in example 1, with the exception that DMAEMA was replaced by MAA.

Again, microparticles with an equilibrium water content was 70 wt.-% were obtained. The mean volume diameter was 8.3 µm, and about 90 % of the particles were smaller than 12.5 µm.

### Example 3: Preparation of gels comprising anionically and cationically charged microparticles.

The lyophilized microparticles prepared according to examples 1 and 2 were separately dispersed in Hepes buffer (100 mM pH 7). From each type of microparticle, dispersions with a solid content of 10, 15, and 25 wt.-%, were prepared. The dispersions were stored at 4°C for 2 hours to allow full hydration of the microspheres. With a solid content of 10 %, the dispersions were free flowing, whereas the 25 % dispersions had a very high viscosity. Subsequently, equal volumes (200 µL) of dispersions with oppositely charged microspheres, but with the same solid content, were mixed using a positive displacement pipette. Gelation was observed to occur instantly upon mixing.

### Example 4: Rheological properties of gels comprising anionically and cationically charged microparticles

The viscoelastic properties of the gel prepared according to example 3 having a solid content of 15 wt.-% were investigated in a controlled strain experiment, using a controlled stress rheometer (AR1000-N, TA Instruments), equipped with a 20 mm acrylic flat plate geometry and a gap of 500 µm. Immediately after gel formation, the sample was placed between the plates. A solvent trap was used to prevent evaporation of the solvent. The viscoelastic properties of the sample were determined by measuring the G' (shear storage modulus) and G" (loss modulus) at 20°C with a constant strain of 1 % and constant frequency of 1 Hz. Frequency sweep and strain sweep experiments were also performed.

In result, the storage modulus (G') increased gradually in time (to ±500 Pa), while the loss modulus (G") remained very low (±30 Pa). The G"/G' ratio or tan(δ) was lower than 0.1, which indicates that the obtained gel is mainly elastic.

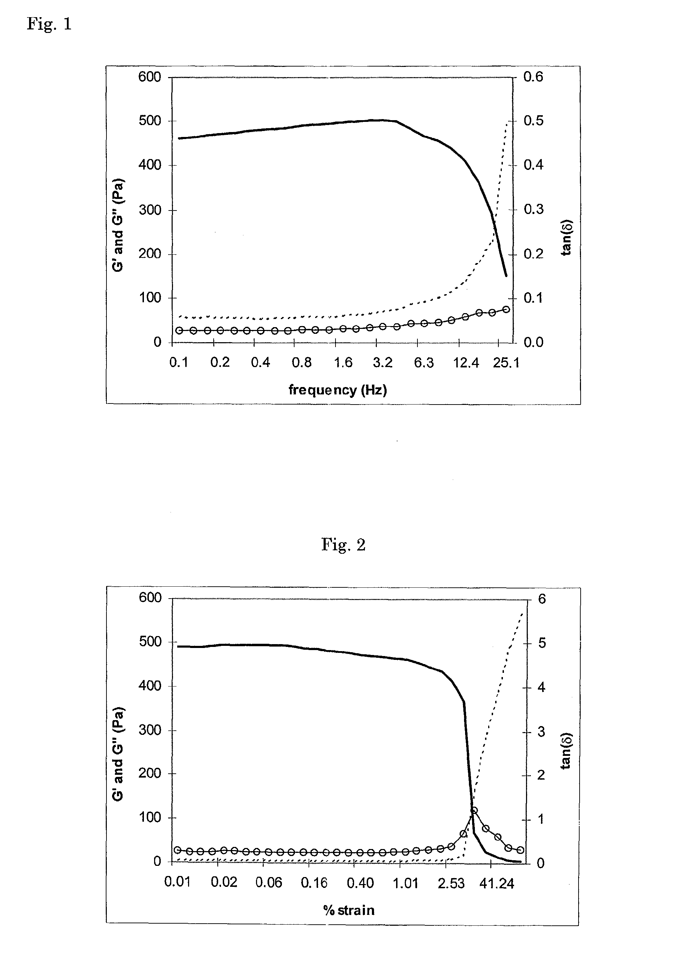

Figure 1 shows the storage modulus G' (―), Loss modulus G" (∞∞) and tan(δ) (---) of the gel at 20°C as a function of the oscillation frequency.

Figure 2 shows the storage modulus G' (―), Loss modulus G" (∞∞) and tan(δ) (---) of the gel at 20°C as a function of the strain.

Figure 3 shows the storage modulus G' (―), Loss modulus G" (∞∞) and tan(δ) (---) of the gel at 20°C as a function of the time.

Creep experiments were performed to evaluate the recovery capacity of the gel network after deformation. A constant stress of 1 Pa and frequency of 1 Hz were applied while the strain was monitored. After 60 seconds, the stress was removed and the recovery of the original network structure was monitored during 2 minutes.

During retardation (i.e. the deformation step), the gel responded to the applied stress (1 Pa), evolving to 0.15 % strain. When the stress was removed, the percentage strain immediately dropped to 0.04 %, confirming the almost fully elastic properties of the network.

To determine the yield point of the gel, stress sweep experiments were performed at 20°C. During these experiments, the G', G" and tan(δ) were monitored while the stress was increased. The frequency was kept constant to 1 Hz. The experiment was performed 4 times in a row using the same sample. After each experiment the sample was allowed to recover for 1 hour.

In result, the G' decreased and the tan(δ) increased while the stress was gradually increased. When the applied stress reached 10 Pa, the G' dramatically dropped from 300 Pa to 3 Pa, whereas the tan(δ) increased from 0.08 to 5.55. After 1 hour, the same experiment was repeated and resulted in a similar plot. Four stress sweep experiments were performed in total, each giving comparable values for G', G" and tan(δ).

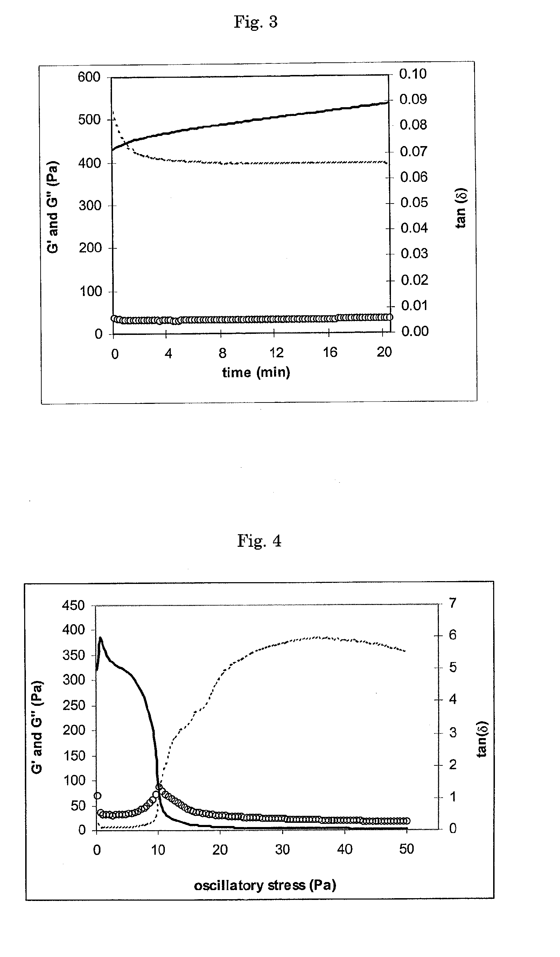

Figure 4 shows clearly that the gel starts to flow when the applied stress exceeds 10 Pa. This behaviour is reversible as demonstrated by the fact that the experiment could be repeated several times. This indicates that after a certain time the ionic interactions between the microspheres are reestablished and the gel network is rebuilt. In other words, the gel showed typical plastic behaviour with a yield point of 10 Pa.

### Example 5: Preparation of protein-loaded gels comprising anionically and cationically charged microparticles.

Gels were prepared from the oppositely charged microparticles described in examples 1 and 2, and loaded with either lysozyme or bovine serum albumin (BSA) as model protein.

Protein stock solutions of 25 mg/mL were separately prepared from lysozyme and BSA in HEPES buffer (pH 7, 100 mM).

In a first series of experiments, 31.25 mg freeze-dried, positively charged dex-HEMA-DMAEMA microparticles prepared according to example 1 were added to 100 µL protein stock solution (separately for both proteins) and 119 µL HEPES buffer (pH 7, 100 mM). Similarly, negatively charged dex-HEMA-MAA microparticles prepared according to example 2 were loaded with lysozyme or BSA. The microparticles were hydrated for 1 h at 4°C.

In another series of experiments, 62.50 mg freeze-dried cationic or anionic microparticles prepared according to examples 1 and 2 were separately added to 100 µL of either protein stock solution and 88 µL HEPES buffer (pH 7, 100 mM). Again, the microparticles were hydrated for 1 h at 4°C.

Subsequently, the hydrated dispersions of the dex-HEMA-DMAEMA and dex-HEMA-MAA microparticles which contained the same protein and has the same particle content were thoroughly mixed and then transferred into Eppendorf cups. Upon mixing, a gel was formed as coherent sediment at the bottom of the Eppendorf cup. The mixing of the oppositely charged microparticle dispersions obtained in the first series yielded gels with a solid content of about 12.5%, whereas the second series led to gels with a solid content of about 25%.

### Example 6: Protein release from gels comprising anionically and cationically charged microparticles.

To each of the gels prepared according to example 5, 1.5 mL release buffer (HEPES, pH 7, 100 mM, isotonised with NaCl) was added. Subsequently, the cups were incubated at 37°C. Samples of 1.0 mL were taken at various intervals and replaced by 1.0 mL fresh buffer. The protein concentration in the samples was measured colorimetrically using the BCA Protein Assay.

In result, relatively smooth release profiles were obtained for all gels. The gels with a higher solid content (25%) showed a slower release than those with the lower content (12.5%). Furthermore, BSA was released more slowly than lysozyme, perhaps due to the higher molecular weight of BSA. The time to reach a release of 50 % of lysozyme was about 20 h for the 12.5%-gel and about 85 h for the 25.5%-gel. For BSA, the values were about 30 h and about 100 h, respectively.

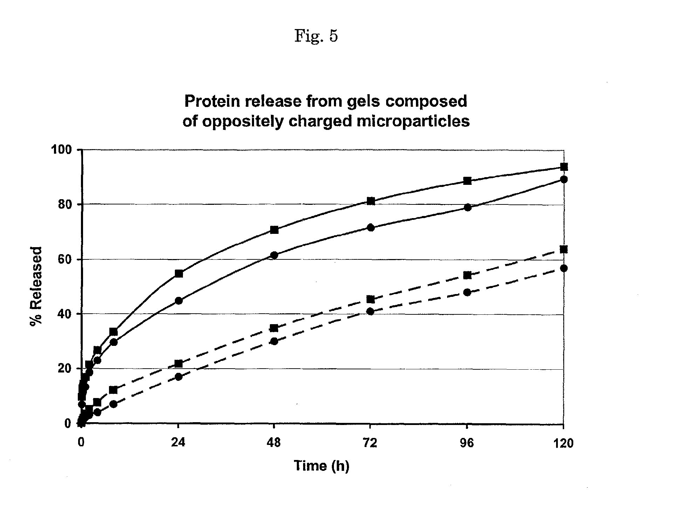

Figure 5 shows the release profiles obtained in example 6. The dotted lines (---) show the protein release from gels with a high solid content (25 %), the solid lines (―) that from gels with a low solid content (12.5%). Solid squares (■) indicate lysozyme release, and solid circles (•) show BSA release.

## Claims

1. An aqueous gel composition comprising a first type of microparticles comprising a cationic polymeric material, and a second type of microparticles comprising an anionic polymeric material.

2. The gel composition of claim 1, further comprising an active compound.

3. The gel composition of claim 1 or 2, wherein the microparticles are of a chemical or physical hydrogel.

4. The gel composition of claim 3, wherein the hydrogel is at least partially composed of a covalently cross-linked polymer which is hydrolysable under physiological conditions.

5. The gel composition of claim 4, wherein the covalently cross-linked polymer is a dextran derivative.

6. The gel composition of any of the preceding claims, further comprising a third type of microparticles, which are essentially neutral.

7. The gel composition of any of the preceding claims, wherein at least one of the types of microparticles is loaded with the active compound.

8. The gel composition of any of the preceding claims, wherein the microparticles have a weight average diameter from about 1 to about 50 µm.

9. The gel composition of any of the preceding claims, having a microparticle content from about 5 to about 50 wt.-%.

10. The gel composition of any of the preceding claims, wherein the microparticles have slow or controlled release characteristics.

11. The gel composition of any of the preceding claims, having viscoelastic properties with a yield point of about 5 to about 300 Pa.

12. The gel composition of any of the preceding claims, wherein the active compound is selected from the group consisting of peptides, proteins, vaccines, nucleotides, hormones, cytostatic or cytotoxic agents, and agents acting on the central nervous system.

13. The gel composition of any of claims 1 to 11, wherein the active compound is a living cell or a plurality of living cells.

14. The gel composition of any of the preceding claims, being adapted for parenteral administration.

15. An aqueous gel composition comprising an active compound and ionically charged polymeric microparticles, wherein the microparticles are suspended in a solution or dispersion of an ionic polymer whose charge is opposite to that of the microparticles.

16. A kit for the preparation of the aqueous gel composition of any of the preceding claims, comprising a dry solid component and an aqueous liquid for reconstituting the gel composition, wherein the solid component comprises the active compound.

17. The kit of claim 16, wherein the solid component further comprises microparticles of the cationic and/or the anionic type.

18. A method for making an aqueous gel, comprising the step of combining positively charged microparticles with negatively charged microparticles in the presence of water.

19. A method for making an aqueous gel, comprising the step of combining ionically charged microparticles with an ionic polymer whose charge is opposite to that of the microparticles, in the presence of water.

20. Use of the aqueous gel composition of any of claims 1 to 15 as slow release carriers for the parenteral delivery of active compounds.

21. Use of the aqueous gel composition of any of claims 1 to 15 as scaffold for tissue engineering.
